# EUROPEAN PATENT APPLICATION

(11) **EP 0 978 275 A1**
(43) Date of publication of application: **09.02.2000**
(21) Application number: 98401998.4
(22) Date of filing: 05.08.1998
(51) Int. Cl.: A61K 9/28

(54) **Solid pharmaceutical dosage form with controlled drug release**

(71) Applicant: Pharma Pass SA, Les Scientifiques, 67400 Illkirch (FR)
(72) Inventor: The designation of the inventor has not yet been filed
(74) Representative: Desrousseaux, Grégoire Marie

(57) **Abstract**

The invention relates to a pharmaceutical dosage form comprising a core containing at least part of an active ingredient, and a functional film coating ensuring controlled onset of the release of the active ingredient contained in the core. The film coating comprises at least one excipient ensuring gastro-resistance and one substance creating an acidic pH in the functional film coating.

The invention allows a lag time between the administration of the pharmaceutical dosage form and the beginning of the release of the drug, said lag time being adjustable to a predefined duration by appropriate formulation. As an example of therapeutic application, the invention provides a dosage form that may release its active ingredient a few hours after administration and is of great interest for reducing some nocturnal occurring diseases, such as ischemic heart attack, asthma or arthritis.

## Description

The invention relates to the field of pharmaceutical dosage forms. More specifically, it relates to a pharmaceutical dosage form, and to a film composition for a pharmaceutical dosage form.

Prior art can be found in gastro-resistant dosage forms. In these formulations, onset of drug release is achieved through a pH change along the GI tract (from acidic in the stomach to alkaline in the gut). However, the onset of drug release is controlled by factors which are essentially independent on the formulation itself. Little work can be found in the literature about tablets or capsules with onset of release independent of external factors. What is described in the present patent is a totally new coating formulation that can be used either on tablets, capsules or pellets and that can achieve a well-controlled onset of active substance release independently from core composition and suitable for tablets as well as capsules or pellets.

In the present context, controlled onset of the drug release means a lag time between the administration of the pharmaceutical dosage form and the beginning of the release of the drug, said lag time being adjusted to a predefined duration by appropriate formulation.

As an example of therapeutic application, a dosage form that could release its active ingredient a few hours after administration would be of great interest for reducing some nocturnal occurring diseases, such as ischemic heart attack, asthma or arthritis. It may be obtained thanks to the invention.

More specifically, according to the invention, there is provided, a pharmaceutical dosage form comprising a core containing at least part of an active ingredient, and a functional film coating ensuring controlled onset of the release of the active ingredient contained in the core.

In one embodiment of the invention, the functional film coating comprises at least one excipient ensuring gastro-resistance and at least one substance creating an acidic pH in the functional film coating.

Preferably, the core is a tablet, a capsule or a pellet. These are solid dosage forms. The invention may generally be applied to all solid dosage forms.

In one embodiment, the pharmaceutical dosage form further comprises an internal coating separating the core from the functional film coating.

This internal coating is preferably a coating of instant release type.

In an embodiment of the invention, the excipient ensuring gastro-resistance is a methacrylic acid copolymer or a cellulose ester.

This excipient ensuring gastro-resistance may be in the amount of 10 to 70% by weight of the whole functional film coating.

In another embodiment of the invention, the substance creating an acidic pH in the functional film coating is comprised in the list of fumaric acid, ascorbic acid, citric acid, tartaric acid, succinic acid.

Preferably, the substance creating an acidic pH in the functional film coating is in the amount of 10 to 80 % by weight of the whole functional film coating.

The functional film coating preferably represents 5 to 25 % of the whole dosage form final weight.

It is also possible that the substance creating an acidic pH in the functional film coating is dispersed into said functional film coating.

The substance creating an acidic pH in the functional film coating preferably has a particle average size of 4 to 60 µm.

The invention also provides a composition for coating a pharmaceutical dosage form, comprising at least one excipient ensuring gastro-resistance and at least one substance creating an acidic pH in the composition.

Preferably, the excipient ensuring gastro-resistance is a methacrylic acid copolymer or a cellulose ester.

In one embodiment, the excipient ensuring gastro-resistance is in the amount of 10 to 70% by weight of the composition.

In another embodiment, the substance creating an acidic pH is comprised in the list of fumaric acid, ascorbic acid, citric acid, tartaric acid and succinic acid.

This substance creating an acidic pH is preferably in the amount of 10 to 80% by weight of the composition.

Advantageously, the substance creating an acidic pH is dispersed into said composition. It may have a particle average size of 4 to 60 µm.

Different embodiments of the invention will now be described, by way of non-limiting examples, with reference to the accompanying drawing, which shows a graph of per cent of release of active ingredient as a function of time.

The dosage form of the invention can be a tablet, a capsule or a pellet. In every case, it may comprise two parts:
- an internal part hereinafter referred as the core
- an external part hereinafter referred as the coating

The core may be produced by well-established conventional techniques of tablet, capsules and pellet manufacturing. Examples of these can be found in numerous papers and books, e.g. Baker GS, Rhodes CR: Modern Pharmaceutics. Third edition. ISBN 0-8247-9371-4. publisher: Marcel Dekker, New York, Basle, (1996). Formulation of these cores comprises immediate release products as well as modified release dosage forms.

The cores are then coated. Composition of the coating is designed to achieve a delayed onset of drug release. Formulation of this coating comprises an excipient traditionally used to get gastro-resistance. This excipient can be but is not restricted to methacrylic acid copolymer, type C or cellulose acetate phtalate or hydroxypropyl methylcellulose phtalate as described in the US National Formulary. Manufacturers of such substances include but are not restricted to Röhm Pharma, Darmstadt, Germany, Shin Etsu, Tokyo, Japan or Eastman Chemicals, Kingsport (Te), USA. When used in conventional formulation, as described in the literature, these excipients provide only gastro-resistance because they are not soluble in acidic conditions found in the stomach. Once in the duodenum and because of the higher pH in this area, the coating starts dissolving and the release occurs. However, in this case, the drug release is dependent on the local pH in the gastrointestinal tract.

What we have found is that in case these gastro-resistant excipients are associated with an acidic substance in the same coating, the solubilization of the coating becomes independent on the external pH conditions. Suitable acidic substances for this application include any acidic substance available as a powder. The preferred acidic substances include (without being restricted to) those described in major pharmacopoeias, as for example ascorbic acid, tartaric acid, citric acid or preferably fumaric acid or succinic acid. The acidic product included in the coating produces a local acidic pH in the coating layer and these acidic conditions keep the coating intact whatever the external pH conditions in the gastrointestinal tract. However, the acidic substance diffuses slowly through the coating, and after a certain period of time disappears totally from the coating. Now depending on the external pH conditions (alkaline in the intestine), the coating is then dissolved and the drug can be released. A preferred way of preparing the coating is as follows: The gastroresistant excipient is an aqueous suspension directly available from the suppliers; for instance, it may be Eudragit L 30D-55 available from Roehm Pharma Germany, Aquacoat CPD, available from FMC (USA) or Eastacryl 30D, available from Eastman (USA). The gastroresistant excipient is mixed with the plasticizer. Sufficient time and stirring are allowed to get a good contact between these excipients, usually at least 30 minutes. The acidic substance is suspended in water. The acidic substance is preferably micronized before being used, particle size being in the range 4 to 60 µm. An antiadherent excipient (micronized talc or silicon dioxide) can also be added to the suspension of acidic product, in order to limit tablet sticking. The two suspension are then mixed and kept under gentle stirring, while being sprayed onto the dosage forms. This process can be performed either in a fluidized bed device as those manufactured by Glatt (Germany) or Aeromatic (Switzerland) or in a coating pan (conventional or preferably perforated) as those manufactured by Driam (Germany), Manesty (United Kingdom), Accela Cota (Italy) or Vector (USA).

Several examples are now detailed.

### Example 1: tablets of verapamil hydrochloride

In this example, an intermediate coating is applied between the core and the acidic coating, in order to protect the acidic coating from the influence of the drug which is itself acidic (hydrochloride).

### Tablet cores

A tablet core of verapamil hydrochloride is prepared according to the following formulation:

| Ingredient | Amount per dosage form (mg) |
|---|---|
| Verapamil (hydrochloride) | 240.00 |
| Avicel ph 302 | 66.00 |
| Povidone (Plasdone K-90) | 4.00 |
| Polyethylene oxide (Polyox N-750) | 40. 00 |
| Polyethylene oxide (Polyox 1105) | 40.00 |
| Aerosil 200 | 4.00 |
| Magnesium stearate | 4.00 |
| Purified water | 26.00 |
| Isopropyl alcohol | 10.00 |

Plasdone is dissolved in isopropyl alcohol - water. The mixture of verapamil hydrochloride, Avicel and Polyox is granulated with the above solution. Wet granules are forced through a sieve (1.6mm mesh size) and dried in a fluidized bed (45°C during 45 minutes). Dried granules are calibrated by forcing though a sieve 0.8 mm mesh size. Magnesium stearate and Aerosil 200 are mixed with the granules and the mixture is pressed into tablets 11 mm diameter with 11 mm curvature.

### First coating

| Ingredient | Amount per dosage form (mg) |
|---|---|
| Tablet core | 400.00 |
| Hydroxypropyl-methylcellulose (Pharmacoat 603) | 20.00 |
| Polyethylene glycol (carbowax 1450) | 3.00 |
| Sodium phosphate tribasic | 2.00 |
| Purified water | 200.00 |

Tribasic sodium phosphate, PEG and Pharmacoat 603 are dissolved in water. The resulting solution is sprayed onto the tablets in a coating pan (Vector LCDS), with the following process parameters

| | |
|---|---|
| Air flow (m³/h) | 100-110 m3/h |
| Liquid flow (g/min) | 6-7 g/min |
| Inlet temperature | 50°C |
| Spraying pressure | 2.8 bar |

### Second coating

| Ingredient | Amount per dosage form (mg) |
|---|---|
| Tablet | 425.00 |
| Methacrylic acid copolymer C, aqueous dispersion (Eudragit L30D-55) | 27.00 |
| Fumaric acid | 21.00 |
| Triethyl citrate | 3.60 |
| Talc | 8.40 |
| Purified water | 290.00 |

Triethyl citrate is dissolved in 40% water. Eudragit is added and stirred 45 minutes. Talc and fumaric acid are suspended in the remaining part of water. The suspensions are mixed and the mixture is sprayed onto the tablets in a coating pan (Vector LCDS), with the following coating parameters:

| | |
|---|---|
| Air flow (m³/h) | 100-110 m3/h |
| Liquid flow (g/min) | 6-7 g/min |
| Inlet temperature | 35°C |
| Spraying pressure | 2.8 bar |

The final tablets is tested in dissolution with USP23 method 〈711〉, apparatus 1, with the following operating conditions:

| | |
|---|---|
| Rotation speed | 100 rpm |
| Dissolution medium | buffer pH 6.8, 900 ml |
| Temperature | 37°C |
| Assay | UV, on-line, 254 nm |

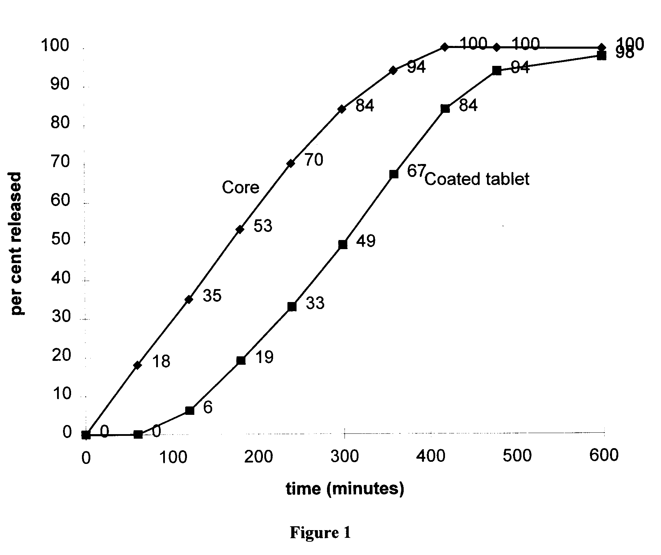

Average results are reported in the graph of figure 1, that shows the per cent release as a function of time in minutes, for the core and for the coated tablet.

### Example 2: tablets of diltiazem

### Tablet cores

A tablet core of diltiazem hydrochloride is prepared according to the following formulation:

| Ingredient | Amount per dosage form (mg) |
|---|---|
| Diltiazem (hydrochloride) | 240.00 |
| Povidone (Plasdone K29-32) | 4.00 |
| Polyethylene oxide (Polyox 301) | 200.00 |
| Aerosil 200 | 3.00 |
| Magnesium stearate | 3.00 |
| Purified water | 20.00 |
| Isopropyl alcohol | 56.00 |

Plasdone is dissolved in isopropyl alcohol - water. The mixture of diltiazem hydrochloride and Polyox is granulated with the above solution. Wet granules are forced through a sieve (1.6mm mesh size) and dried in an oven (45°C, overnight). Dried granules are calibrated by forcing through a sieve 0.8 mm mesh size. Magnesium stearate and Aerosil 200 are mixed with the granules and the mixture is pressed into tablets 11 mm diameter with 11 mm curvature.

### First coating

| Ingredient | Amount per dosage form (mg) |
|---|---|
| Tablet core | 440.00 |
| Hydroxypropyl-methylcellulose (Pharmacoat 603) | 20.00 |
| Polyethylene glycol (carbowax 1450) | 3.00 |
| Sodium phosphate tribasic | 2.00 |
| Purified water | 200.00 |

Tribasic sodium phosphate, PEG and Pharmacoat 603 are dissolved in water. The resulting solution is sprayed onto the tablets in a coating pan (Vector LCDS), with the following process parameters

| | |
|---|---|
| Air flow (m³/h) | 100-110 m3/h |
| Liquid flow (g/min) | 6-7 g/min |
| Inlet temperature | 50°C |
| Spraying pressure | 2.8 bar |

### Second coating

| Ingredient | Amount per dosage form (mg) |
|---|---|
| Tablet | 425.00 |
| Methacrylic acid copolymer C, aqueous dispersion (Eudragit L30D-55) | 27.00 |
| Fumaric acid | 21.00 |
| Triethyl citrate | 3.60 |
| Talc | 8.40 |
| Purified water | 290.00 |

Triethyl citrate is dissolved in 40% water. Eudragit is added and stirred 45 minutes. Talc and fumaric acid are suspended in the remaining part of water. The suspensions are mixed and the mixture is sprayed onto the tablets in a coating pan (Vector LCDS), with the following coating parameters:

| | |
|---|---|
| Airflow | 100-110m3/h |
| Liquid flow | 6-7 g/min |
| Inlet temperature | 35°C |
| Spraying pressure | 2.8 bar |

### Example 3: pellets of diazepam

### Pellet cores

The following formulation of pellet cores is prepared:

| **Ingredient** | **Amount per dosage unit (mg)** |
|---|---|
| Diazepam (micronized) | 5.00 |
| Sugar non-pareils 600 µm | 200.00 |
| Plasdone K 29-32 | 10.00 |
| Water | 100.00 |

Plasdone is dissolved in water. Diazepam is dispersed into the Plasdone solution and homogenized with Ultra Turrax homogenizer. The suspension is sprayed onto the pellets in a GPCG1 Glatt device fitted with a Würster chamber, with the following process parameters:

| | |
|---|---|
| Fluidized bed air volume | 100 m3/h |
| Inlet temperature | 55°C |
| Product temperature | 35°C |
| Pump flow | 13g/min |

### Sub coating

A sub coating is applied onto the pellets:

| Ingredient | Amount per dosage form (mg) |
|---|---|
| Pellets with active | 215.00 |
| Hydroxypropyl-methylcellulose (Pharmacoat 606) | 9.00 |
| Polyethylene glycol 6000 | 1.00 |
| Purified water | 100.00 |

This subcoating is sprayed onto the pellets in a Glatt GPCG1 device fitted with a Würster chamber. the following spraying parameters are used.

### Functional coating

Pellets are coated with the following coating:

| Ingredient | Amount per dosage form (mg) |
|---|---|
| Pellets with active | 225.00 |
| Cellulose acetate phtalate aqueous dispersion (Aquacoat CPD) | 20.00 |
| Fumaric acid | 15.00 |
| Triethyl citrate | 4.00 |
| Talc | 3.00 |
| Purified water | 100.00 |

Triethyl citrate is dissolved in 40% of water. Aquacoat CPD is added and stirred 45 minutes. Talc and fumaric acid are suspended in the remaining part of water. The suspensions are mixed and the mixture is sprayed onto the tablets in a coating pan (Vector LCDS), with the following coating parameters:

| | |
|---|---|
| Air flow | 100-110m3/h |
| Liquid flow | 6-7 g/min |
| Inlet temperature | 35°C |
| Spraying pressure | 2.8 bar |

Capsules size 2 are then filled with the coated pellets.

### Example 4: immediate release tablets of diazepam

### Tablet cores

Tablet cores of diazepam are prepared according to the following formulation:

| Ingredient | Amount per dosage form (mg) |
|---|---|
| Diazepam | 5.00 |
| Avicel PH101 | 100.00 |
| Povidone (Plasdone K-29-32) | 4.00 |
| Polyplasdone XL | 5.00 |
| Magnesium stearate | 0.50 |
| Aerosil 200 | 0.50 |
| Water | 18.00 |
| Isopropyl alcohol | 3.00 |

Povidone is dissolved in isopropanol/water. Diazepam and Avicel PH101 are mixed and granulated with the povidone solution. Granules are forced through a screen 1.6mm mesh size and dried in a fluidized bed (45°C and 60 minutes). Dried granules are passed through a sieve 0.8 mm mesh size. Magnesium stearate and Aerosil 200 are mixed to the granules and the resulting mixture is pressed into tablet 6 mm diameter and 6 mm curvature radius.

### Coating

| Ingredient | Amount per dosage form (mg) |
|---|---|
| Tablet | 115.00 |
| Methacrylic acid copolymer C, aqueous dispersion (Eudragit L30D-55) | 10.00 |
| Fumaric acid | 7.00 |
| Triethyl citrate | 1.00 |
| Talc | 2.00 |
| Purified water | 60.00 |

Triethyl citrate is dissolved in 40% of water. Eudragit is added and stirred 45 minutes. Talc and fumaric acid are suspended in the remaining part of water. The suspensions are mixed and the mixture is sprayed onto the tablets in a coating pan (Vector LCDS), with the following coating parameters:

| | |
|---|---|
| Air flow | 100-110 m3/h |
| Liquid flow | 6-7 g/min |
| Inlet temperature | 38°C |
| Spraying pressure | 2.8 bar |

### Example 5: verapamil bilayer tablets, immediate release + sustained release

Verapamil bilayer cores are prepared according to the following formulation:

| Ingredient (sustained release part) | Amount per dosage form (mg) |
|---|---|
| Verapamil (hydrochloride) | 180.00 |
| Avicel ph 302 | 50.00 |
| Povidone (Plasdone K-90) | 4.00 |
| Polyethylene oxide (Polyox N-750) | 40.00 |
| Polyethylene oxide (Polyox 1105) | 40.00 |
| Aerosil 200 | 4.00 |
| Magnesium stearate | 4.00 |
| Purified water | 22.00 |
| Isopropyl alcohol | 8.00 |

Plasdone is dissolved in isopropyl alcohol - water. The mixture of verapamil hydrochloride, Avicel and Polyox is granulated with the above solution. Wet granules are forced through a sieve (1.6mm mesh size) and dried in a fluidized bed (45°C during 45 minutes). Dried granules are calibrated by forcing through a sieve 0.8 mm mesh size. Magnesium stearate and Aerosil 200 are mixed with the granules

| Ingredient (immediate release part) | Amount per dosage form (mg) |
|---|---|
| Verapamil (hydrochloride) | 60.00 |
| Avicel ph101 | 50.00 |
| Plasdone K29-32 | 3.00 |
| Polyplasdone XL | 6.00 |
| Aerosil 200 | 0.50 |
| Magnesium stearate | 0.50 |
| Purified water | 13.00 |
| Isopropyl alcohol | 5.00 |

Plasdone is dissolved in isopropyl alcohol - water. The mixture of verapamil hydrochloride, and Avicel is granulated with the above solution. Wet granules are forced through a sieve (1.6mm mesh size) and dried in a fluidized bed (45°C during 45 minutes). Dried granules are calibrated by forcing through a sieve 0.8 mm mesh size. Magnesium stearate, Polyplasdone XL and Aerosil 200 are mixed with the granules.

Immediate release and sustained release granules are pressed into bilayer tablets 12 mm with a twin feeder press.

### First coating

| Ingredient | Amount per dosage form (mg) |
|---|---|
| Tablet core | 442.00 |
| Hydroxypropyl-methylcellulose (Pharmacoat 603) | 20.00 |
| Polyethylene glycol (carbowax 1450) | 3.00 |
| Sodium phosphate tribasic | 2.00 |
| Purified water | 200.00 |

Tribasic sodium phosphate, PEG and Pharmacoat 603 are dissolved in water. The resulting solution is sprayed onto the tablets in a coating pan (Vector LCDS), with the following process parameters

| | |
|---|---|
| Airflow | 100-110m3/h |
| Liquid flow | 6-7 g/min |
| Inlet temperature | 50°C |
| Spraying pressure | 2.8 bar |

### Second coating

| Ingredient | Amount per dosage form (mg) |
|---|---|
| Tablet | 467.00 |
| Methacrylic acid copolymer C, aqueous dispersion (Eudragit L30D-55) | 27.00 |
| Fumaric acid | 21.00 |
| Triethyl citrate | 3.60 |
| Talc | 8.40 |
| Purified water | 290.00 |

Triethyl citrate is dissolved in 40% of water. Eudragit is added and stirred 45 minutes. Talc and fumaric acid are suspended in the remaining part of water. The suspensions are mixed and the mixture is sprayed onto the tablets in a coating pan (Vector LCDS), with the following coating parameters:

| | |
|---|---|
| Air flow | 100-110 m3/h |
| Liquid flow | 6-7 g/min |
| Inlet temperature | 35°C |
| Spraying pressure | 2.8 bar |

### Example 6: diltiazem bilayer tablets, immediate release + sustained release

### Tablet cores

Diltiazem bilayer cores are prepared according to the following formulation:

| Ingredients (sustained release pad) | Amount per dosage form (mg) |
|---|---|
| Diltiazem (hydrochloride) | 180.00 |
| Povidone (Plasdone K29-32) | 4.00 |
| Polyethylene oxide (Polyox 301) | 200.00 |
| Aerosil 200 | 3.00 |
| Magnesium stearate | 3.00 |
| Purified water | 16.00 |
| Isopropyl alcohol | 50.00 |

Plasdone is dissolved in isopropyl alcohol - water. The mixture of diltiazem hydrochloride and Polyox is granulated with the above solution. Wet granules are forced through a sieve (1.6mm mesh size) and dried in an oven (45°C, overnight). Dried granules are calibrated by forcing through a sieve 0.8 mm mesh size. Magnesium stearate and Aerosil 200 are mixed with the granules.

| Ingredients (immediate release part) | Amount per dosage form (mg) |
|---|---|
| Diltiazem (hydrochloride) | 60.00 |
| Povidone (Plasdone K29-32) | 4.00 |
| Avicel PH101 | 123.00 |
| Aerosil 200 | 1.00 |
| Magnesium stearate | 2.00 |
| Purified water | 45.00 |

Plasdone is dissolved in water. Diltiazem hydrochloride and Avicel mixture is granulated with the above solution. Wet granules are forced through a sieve (1.6mm mesh size) and dried in an oven (45°C, overnight). Dried granules are calibrated by forcing through a sieve 0.8 mm mesh size. Magnesium stearate and Aerosil 200 are mixed with the granules.

Immediate release and sustained release granules are pressed into bilayer tablets 12 mm diameter and 12 mm curvature radius with a twin feeder press.

### Subcoating

In this example, an intermediate coating is applied between the core and the acidic coating, in order to protect the acidic coating from the influence of the drug which is itself acidic (hydrochloride).

| Ingredient | Amount per dosage form (mg) |
|---|---|
| Tablet core | 580.00 |
| Hydroxypropyl-methylcellulose (Pharmacoat 603) | 20.00 |
| Polyethylene glycol (carbowax 1450) | 3.00 |
| Sodium phosphate tribasic | 2.00 |
| Purified water | 200.00 |

Tribasic sodium phosphate, PEG and Pharmacoat 603 are dissolved in water. The resulting solution is sprayed onto the tablets in a coating pan (Vector LCDS), with the following process parameters

| | |
|---|---|
| Air flow | 100-110 m3/h |
| Liquid flow | 6-7 g/min |
| Inlet temperature | 50°C |
| Spraying pressure | 2.8 bar |

### Functional coating

| Ingredient | Amount per dosage form (mg) |
|---|---|
| Tablet | 605.00 |
| Methacrylic acid copolymer C, aqueous dispersion (Eudragit L30D-55) | 33.00 |
| Fumaric acid | 27.00 |
| Triethyl citrate | 5.00 |
| Talc | 10.00 |
| Purified water | 290.00 |

Triethyl citrate is dissolved in 40% water. Eudragit is added and stirred 45 minutes. Talc and fumaric acid are suspended in the remaining part of water. The suspensions are mixed and the mixture is sprayed onto the tablets in a coating pan (Vector LCDS), with the following coating parameters:

| | |
|---|---|
| Air flow | 100-110 m3/h |
| Liquid flow | 6-7 g/min |
| Inlet temperature | 35°C |
| Spraying pressure | 2.8 bar |

### Example 7: external cosmetic coating

Formulation of core and coatings is the same than in example 1. However, in order to allow tablet identification, a third coating is used. This coating contains a pigment and is designed to allow a fast disintegration, independent of pH:

| Ingredient | Amount per tablet (mg) |
|---|---|
| Tablet 1^{st} + 2^{nd} coating | 487.00 |
| Pharmacoat 606 | 10.25 |
| PEG 6000 | 3.00 |
| Iron oxide (yellow) | 0.12 |
| Iron oxide (red) | 0.63 |
| Titanium dioxide | 2.38 |
| Water (purified) | 200.00 |

PEG and Pharmacoat are dissolved in 70% water. Pigments and titanium dioxide are suspended in the remaining part of water and homogenized. The solution and the suspension are mixed and kept under gentle stirring during spraying onto the tablets, in a Vector LCDS coating pan, with the following parameters:

| | |
|---|---|
| Air flow | 100-110 m3/h |
| Liquid flow | 9-10 g/min |
| Inlet temperature | 65°C |
| Spraying pressure | 2.8 bar |

## Claims

1. A pharmaceutical dosage form comprising a core containing at least part of an active ingredient, and a functional film coating ensuring controlled onset of the release of the active ingredient contained in the core.

2. The pharmaceutical dosage form of claim 1, wherein the functional film coating comprises at least one excipient ensuring gastro-resistance and at least one substance creating an acidic pH in the functional film coating.

3. The pharmaceutical dosage form of claim 1 or 2, wherein the core is a tablet, a capsule or a pellet.

4. The pharmaceutical dosage form of claim 1, 2 or 3, further comprising an internal coating separating the core from the functional film coating.

5. The pharmaceutical coating of claim 4, wherein the internal coating is a coating of instant release type.

6. The pharmaceutical dosage form of one of claims 2 to 5, wherein the excipient ensuring gastro-resistance is a methacrylic acid copolymer or a cellulose ester.

7. The pharmaceutical dosage form of one of claims 2 to 6, wherein the excipient ensuring gastro-resistance is in the amount of 10 to 70% by weight of the whole functional film coating.

8. The pharmaceutical dosage form of one of claims 2 to 7, wherein the substance creating an acidic pH in the functional film coating is comprised in the list of fumaric acid, ascorbic acid, citric acid, tartaric acid, succinic acid.

9. The pharmaceutical dosage form of one of claims 2 to 8, wherein the substance creating an acidic pH in the functional film coating is in the amount of 10 to 80 % by weight of the whole functional film coating.

10. The pharmaceutical dosage form of one claims 1 to 9 wherein the functional film coating represents 5 to 25 % of the whole dosage form final weight.

11. The pharmaceutical dosage form according to one of claims 2 to 10, wherein the substance creating an acidic pH in the functional film coating is dispersed into said functional film coating.

12. The pharmaceutical dosage form according to one of claims 2 to 11, wherein the substance creating an acidic pH in the functional film coating, has a particle average size of 4 to 60 µm.

13. A composition for coating a pharmaceutical dosage form, comprising at least one excipient ensuring gastro-resistance and at least one substance creating an acidic pH in the composition.

14. The composition of claim 13, wherein the excipient ensuring gastro-resistance is a methacrylic acid copolymer or a cellulose ester.

15. The composition of claim 13 or 14, wherein the excipient ensuring gastro-resistance is in the amount of 10 to 70% by weight of the composition.

16. The composition of claim 13, 14 or 15, wherein the substance creating an acidic pH is comprised in the list of fumaric acid, ascorbic acid, citric acid, tartaric acid and succinic acid.

17. The composition of claim 13, 14, 15 or 16, wherein the substance creating an acidic pH is in the amount of 10 to 80 % by weight of the composition.

18. The composition of one of claims 13 to 17, wherein the substance creating an acidic pH is dispersed into said composition.

19. The composition of one of claims 13 to 18, wherein the substance creating an acidic pH has a particle average size of 4 to 60 µm.
